# EUROPEAN PATENT APPLICATION

(11) **EP 4 670 666 A1**
(43) Date of publication of application: **31.12.2025**
(21) Application number: 24185082.5
(22) Date of filing: 27.06.2024
(51) Int. Cl.: A61B 34/30, A61B 90/11, A61B 90/00, A61B 90/50, A61M 5/32, A61B 5/00, A61B 17/34, A61B 17/00, G09B 23/00

(54) **AN APPARATUS FOR POSITIONING OR NAVIGATING AT LEAST TWO NEEDLES RELATIVE TO AN OBJECT AND A CORRESPONDING SYSTEM COMPRISING SUCH AN APPARATUS**

(71) Applicant: HEICor Biomedical Interventions GmbH, 69120 Heidelberg (DE)
(72) Inventor: LEUSCHNER, Florian, 69115 Heidelberg (DE); SICKLINGER, Florian, 69120 Heidelberg (DE)
(74) Representative: Ullrich & Naumann PartG mbB

(57) **Abstract**

There is provided an apparatus for positioning or navigating at least two needles (2) relative to an object, comprising at least two holding devices (3) for movably holding the needles (2), wherein the holding devices (3) each comprise an adjusting device (4) for moving the needles (2) independently of each other and relative to the object. Further, a system comprising such an apparatus and an imaging device (7) for monitoring and/or guiding a position of at least one needle (2) in the object and a method for induction of a heart failure in an animal with the use of such an apparatus or with the use of such a system are provided.

## Description

The present invention relates to an apparatus for positioning or navigating at least two needles relative to an object, comprising at least two holding devices for movably holding the needles, wherein the holding devices each comprise an adjusting device for moving the needles independently of each other and relative to the object.

Further, the present invention relates to a system comprising such an apparatus and a method for induction of heart failure, HF, in an animal with the use of such an apparatus or with the use of such a system.

Heart failure remains to be a leading cause of death worldwide. Accordingly, there is an immense scientific and clinical need for a better understanding of acute and long-term changes in HF. The use of rodent myocardial infarction, MI, models is an established pillar of basic research investigating such processes and how to treat them. One of the most used models for induction of HF in mice is the transient ligation of the left anterior descending artery, LAD, during open-chest surgery. Even though investigations in this model have led to numerous crucial findings, LAD ligation is a low-throughput procedure with significant surgery-related mortality. Moreover, intubation, mechanical ventilation and thoracotomy within the procedure cause additional tissue damage and trigger a systemic inflammatory response which can confound the immune response evoked by MI. Additionally, the conventional method with opening of the chest is very time-intensive and low throughput. In addition, it is very stressful for the mouse and requires e.g. intensive analgetic regime after the procedure. All available closed-chest models are still suture- and ligation-based which means that the ligation must be performed manually. This is highly dependent on the skills of the surgeon which can result in low reproducibility and high variability. In addition, since the approach requires two steps, the procedure is even more time-intensive.

It is an object of the present invention to provide an apparatus, a system and a method for an effective and reproducible occlusion, partial or total, of a vessel of an animal by simple means.

In accordance with the invention and according to claim 1, the aforementioned object is accomplished by an apparatus for positioning or navigating at least two needles relative to an object, comprising at least two holding devices for movably holding the needles, wherein the holding devices each comprise an adjusting device for moving the needles independently of each other and relative to the object.

Further and according to claim 13, the aforementioned object is accomplished by a system comprising such an apparatus and an imaging device for monitoring and/or guiding a position of at least one needle in the object.

Further and according to claim 15, the aforementioned object is accomplished by a method for induction of heart failure in an animal, preferably with the use of an apparatus according to any one of claims 1 to 12 or with the use of a system according to claim 13 of 14, comprising the following steps: moving two needles to a coronary vessel of the animal; and occluding the coronary vessel, partial or total, by moving the needles together.

According to the invention it has been recognized that it is possible to solve the aforementioned object by providing a suitable apparatus specifically designed for a very effective and reproducible occlusion, partial or total, of a vessel of an animal. Such an apparatus is designed for allowing navigation of at least two needles. The apparatus comprises at least two holding devices for movably holding the needles, wherein the holding devices each comprise an adjusting device for moving the needles independently of each other and relative to the object. The needles are movable independently of each other and relative to the object, so that a reproducible occlusion of coronary vessels in mice for ischemia/reperfusion injury induction is possible by moving the needles to a coronary vessel of the animal and occlusing the coronary vessel by moving the needles together.

Thus, on the basis of the invention an effective and reproducible occlusion, partial or total, of a vessel of an animal by simple means is provided.

It has to be noted, that the term "needle" is used in this document in a very generic sense, so that the term "needle" also comprises cannulas, hollow needles, and tubes, for example.

For providing a very flexible moving of the needles the adjusting device can be designed for translatory moving of each needle in one, two or three spatial directions.

Further, under consideration of an even more flexible moving of the needles the adjusting device can be designed for rotary moving of each needle about one, two or three axes of rotation.

For providing a simplified moving of the needles the adjusting device can be designed for translatory moving of at least two needles together in one, two or three spatial directions.

Regarding a flexible and comfortable use of the apparatus the adjusting device can comprise an adjusting element or a main manipulator for a coarse moving and an adjusting element or a main manipulator for a fine moving for translatory moving one needle or at least two needles together in at least one spatial direction and/or for rotary moving a needle about at least one axis of rotation. Thus, a necessary adjustment of one or more needles can be performed quickly, even if the adjustment comprises a wide translatory and/or rotary movement in a first step and a fine adjustment in a second step.

For providing a compact apparatus which is easy to handle the holding devices can be coupled with or integrated into a base unit or manipulator. Thus, multiple holding devices can be handled together by means of such a base unit or manipulator.

In view of an effective use of the apparatus and a simple handling of the object the apparatus can further comprise an object platform for placement of the object, for example an animal, onto the object platform. The object platform can be easily placed in a necessary position with regard to the base unit or the manipulator.

Within a further embodiment, the apparatus can further comprise a ground platform onto which the holding devices, the base unit, the manipulator and/or the object platform can be positioned. Such a ground platform provides an individual positioning of the holding devices, the base unit, the manipulator and/or the object platform relative to each other.

With regard to a flexible and reliable positioning of the holding devices, the base unit, the manipulator and/or the object platform on the ground platform, the holding devices, the base unit, the manipulator and/or the object platform can be fixed on the ground platform by means of a magnetic stand or magnetic coupling. Such a kind of fixation is flexible and provides a locking of the holding devices, the base unit, the manipulator and/or the object platform by magnetic force. The magnetic force can be individually selected by an individual design of the magnetic stand or magnetic coupling, so that the holding devices, the base unit, the manipulator and/or the object platform can be easily positioned by a user. Alternatively, the base unit or the manipulator can comprise a clamping mechanism for clamping the base unit or the manipulator to the ground platform. The clamping mechanism can comprise a screw mechanism for easily fixing the base unit or the manipulator to the ground platform.

Within a further embodiment the ground platform can comprise a moving device for a translatory moving of the ground platform in a horizontal plane in an x-direction and/or in a y-direction, wherein the moving device can comprise a ground platform manipulator. Such a translatory moving of the ground platform allows relative position change between the ground platform and an imaging device. Since an object platform and a base unit or needle manipulator can be fixed to the ground platform, moving or navigation of the ground platform does not change the position of the needles within an animal during this moving or navigation.

In a further embodiment the apparatus can comprise multiple ground platforms, which can be designed for a side-by-side arrangement of the ground platforms. Thus, multiple ground platforms can easily be used together or in a platform-after-platform mode.

The ground platform can be designed in a size which allows multiple holding devices, base units, manipulators and/or object platforms to be positioned on the ground platform. Thus, an effective and time-saving use of the apparatus is possible.

The imaging device for monitoring and/or guiding a position of at least one needle in the object can comprise an ultrasound transducer, which can be held by a holding equipment.

Exemplary advantages and aspects of embodiments of the present invention are given below:
According to an embodiment a specifically designed manipulator or micromanipulator is provided which allows high-precision navigation of at least two needles in parallel. The manipulator or micromanipulator in combination with a specific object platform and/or ground platform enables reproducible occlusion of coronary vessels in mice for ischemia / reperfusion injury induction.

In order to induce ischemia reperfusion in mice, the left coronary artery can be visualized using an imaging device operating for example with ultrasound. In a minimal-invasive approach, two needles attached to the manipulator or micromanipulator are inserted into the closed chest under image guidance and navigated to the target site of the coronary vessels. Afterwards, the needles are placed either ventral, needle 1, or dorsal, needle 2, of the coronary artery followed by occlusion of the vessel by moving the needles together. The manipulator or micromanipulator allows high-precision navigation of the two needles in all three dimensions inside the chest. Moreover, the angulation of the needles in all dimensions can be modified. These feature are important to guarantee optimal occlusion and adjustments throughout the procedure. After a defined time, which can be up to 2 hours, the needles are removed, for reperfusion.

There are several ways how to design and further develop the teaching of the present invention in an advantageous way. To this end it is to be referred to the following explanation of examples of embodiments of the invention, illustrated by the drawing. In the drawing
Fig. 1 shows a perspective view of an embodiment of an apparatus and system according to the invention,
Fig. 2 shows a perspective view of the apparatus shown in Fig. 1,
Fig. 3 shows a further perspective view of the apparatus shown in Fig. 1 indicating the possibility of a needle rotation about a y-axis,
Fig. 4 shows a further perspective view of the apparatus shown in Fig. 1 indicating the possibility of a needle rotation about a z-axis and
Fig. 5 shows a further perspective view of the apparatus shown in Fig. 1 indicating the possibility of a needle translation in an x-axis and a z-axis.

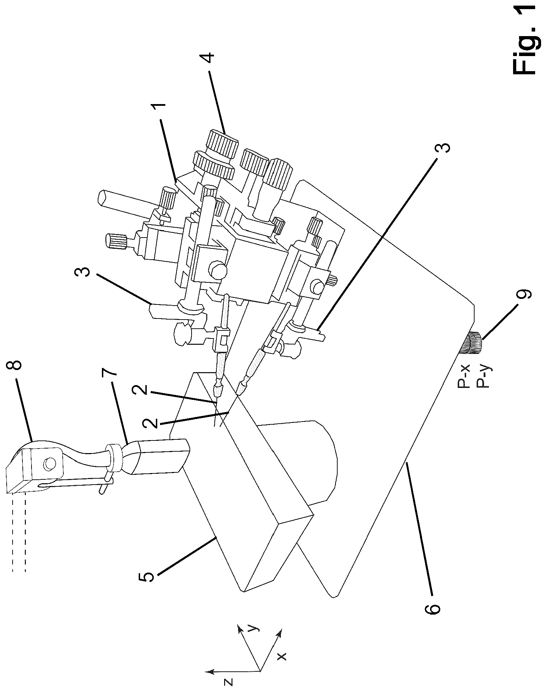

Fig. 1 shows a perspective view of an embodiment of an apparatus in the form of a manipulator 1. The manipulator 1 comprises two needles 2 which are movably held by individual holding devices 3. Each holding device 3 comprises an adjusting device 4 for moving the needles 2 independently of each other and relative to an object platform 5.

The manipulator 1 and the object platform 5 are positioned on a ground platform 6. An imaging device 7 comprising an ultrasound transducer is also positioned on the ground platform 6. An object, for example an animal, can be positioned on the object platform 5. The ground platform 6 can be moved in a horizontal x-y-plane.

The manipulator 1 is a specialized micro-manipulator which allows high-precision navigation of two needles 2. The micro-manipulator enables combined translation of both needles 2 in all three dimensions and isolated navigation of each needle independently, i.e. translation in two dimensions and rotation in two axes.

The object platform 5 is a specialized animal or rodent platform which allows placement of a sedated animal in supine position. Both, the animal or object platform 5 and the manipulator 1 are attached to the ground platform 6. By the use of magnetic stands, the position of the animal or object platform 5 and the manipulator 1 is flexible, but can be locked.

The system includes a holding equipment 8 which allows placement of an imaging device 7 in the form of an ultrasound transducer relative to an object or animal. The holding equipment 8 is not connected to the ground platform 6 and can be positioned / navigated independently from the ground platform 6.

As indicated in Fig. 1, the ground platform 6 can be moved by employment of a platform manipulator 9 or micro-manipulator, P-x for translation on the x-axis and P-y for translation on the y-axis. Navigation of the ground platform 6 allows relative position change between the ground platform 6, including animal or object platform 5 and needle manipulator 1, and the imaging device 7. Since the animal or object platform 5 and the needle manipulator 1 are attached to the ground platform 6, navigation of the ground platform 6 does not change the position of the needles 2 within an object or animal during the intervention.

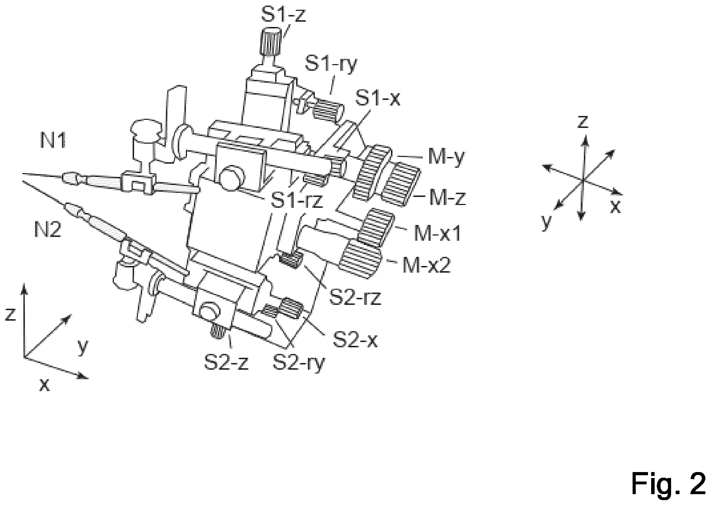

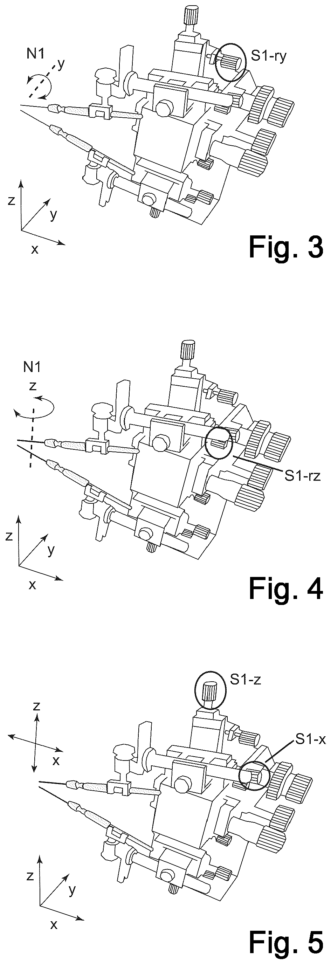

Fig. 2 shows a perspective view of the apparatus shown in Fig. 1 indicating the multiple moving possibilities of the needles 2. Fig. 3-5 show further perspective views of the apparatus shown in Fig. 1, particularly indicating the possibility of a needle rotation about a y-axis and about a z-axis - Fig. 3 and Fig. 4 - and the possibility of a needle translation in an x-axis and a z-axis - Fig. 5.

Fig. 2-5 show Cartesian coordinate axes x, y and z for explaining the possibilities of moving the needles 2.

In the following, the two needles 2 are specified by the reference signs N1 and N2.

Further, the adjusting device 4 comprises adjusting elements in the form of main manipulators M-y, M-z, M-x1 and M-x2. For movement of the needles N1 and N2, the main manipulators M-y, M-z, M-x1 and M-x2 allow translation of both needles N1 and N2 at the same time. M-x1 is for coarse translational movement on the x-axis, whereas M-x2 enables fine adjustment of translational movement on the x-axis.

The adjusting device 4 comprises further adjusting elements in the form of four further manipulators S1-z, S1-x, S1-ry and S1-rz and four further manipulators S2-z, S2-x, S2-ry and S2-rz for movement of each individual needle N1 and N2, respectively.

For movement of needle N1, the following four further manipulators can be adjusted:
S1-z allows translation of needle N1 in the z-axis.
S1-x allows translation of needle N1 in the x-axis.
S1-ry allows rotation of needle N1 around the y-axis, xz-plane.
S1-rz allows rotation of needle N1 around the z-axis, xy-plane.

For movement of needle N2, the following four further manipulators can be adjusted:
S2-z allows translation of needle N2 in the z-axis.
S2-x allows translation of needle N2 in the x-axis.
S2-ry allows rotation of needle N2 around the y-axis, xz-plane.
S2-rz allows rotation of needle N2 around the z-axis, xy-plane.

According to further embodiments of the invention an arrangement of multiple intervention ground platforms 6 side by side is possible. Thereby, a single operator can efficiently utilize waiting times during vessel occlusion and perform interventions in parallel. Embodiments include either arrangement of multiple ground platforms 6 side by side, with an attached animal or object platform 5 and micromanipulator or manipulator 1 on each ground platform 6, or multiple animal or object platforms 5 and micromanipulators or manipulators 1 positioned on one for example big ground platform 6.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### List of reference signs

- 1: manipulator
- 2: needle
- 3: holding device
- 4: adjusting device
- 5: object platform
- 6: ground platform
- 7: imaging device
- 8: holding equipment
- 9: platform manipulator

- M-y: main manipulator
- M-z: main manipulator
- M-x1: main manipulator
- M-x2: main manipulator
- N1: needle
- N2: needle
- S1-z: further manipulator
- S1-x: further manipulator
- S1-ry: further manipulator
- S1-rz: further manipulator
- S2-z: further manipulator
- S2-x: further manipulator
- S2-ry: further manipulator
- S2-rz: further manipulator

## Claims

1. An apparatus for positioning or navigating at least two needles (2) relative to an object, comprising at least two holding devices (3) for movably holding the needles (2), wherein the holding devices (3) each comprise an adjusting device (4) for moving the needles (2) independently of each other and relative to the object.

2. The apparatus according to claim 1, wherein the adjusting device (4) is designed for translatory moving of each needle (2) in one, two or three spatial directions.

3. The apparatus according to claim 1 or 2, wherein the adjusting device (4) is designed for rotary moving of each needle (2) about one, two or three axes of rotation.

4. The apparatus according to any of claims 1 to 3, wherein the adjusting device (4) is designed for translatory moving of at least two needles (2) together in one, two or three spatial directions.

5. The apparatus according to any of claims 1 to 4, wherein the adjusting device (4) comprises an adjusting element or a main manipulator (M-x1) for a coarse moving and an adjusting element or a main manipulator (M-x2) for a fine moving for translatory moving one needle (2) or at least two needles (2) together in at least one spatial direction and/or for rotary moving a needle (2) about at least one axis of rotation.

6. The apparatus according to any of claims 1 to 5, wherein the holding devices (3) are coupled with or integrated into a base unit or manipulator (1).

7. The apparatus according to any of claims 1 to 6, wherein the apparatus further comprises an object platform (5) for placement of the object, for example an animal, onto the object platform (5).

8. The apparatus according to any of claims 1 to 7, wherein the apparatus further comprises a ground platform (6) onto which the holding devices (3), the base unit, the manipulator (1) and/or the object platform (5) can be positioned.

9. The apparatus according to claim 8, wherein the holding devices (3), the base unit, the manipulator (1) and/or the object platform (5) are or is fixed on the ground platform (6) by means of a magnetic stand or magnetic coupling.

10. The apparatus according to claim 8 or 9, wherein the ground platform (6) comprises a moving device for a translatory moving of the ground platform (6) in a horizontal plane in an x-direction and/or in a y-direction, wherein the moving device can comprise a ground platform manipulator (9).

11. The apparatus according to any of claims 8 to 10, wherein the apparatus comprises multiple ground platforms (6), which are designed for a side-by-side arrangement of the ground platforms (6).

12. The apparatus according to any of claims 8 to 11, wherein multiple holding devices (3), base units, manipulators (1) and/or object platforms (5) are positioned on the ground platform (6).

13. A system comprising an apparatus according to one of claims 1 to 12 and an imaging device (7) for monitoring and/or guiding a position of at least one needle (2) in the object.

14. The system according to claim 13, wherein the imaging device (7) comprises an ultrasound transducer, which can be held by a holding equipment (8).

15. A method for induction of a heart failure in an animal, preferably with the use of an apparatus according to any one of claims 1 to 12 or with the use of a system according to claim 13 of 14, comprising the following steps:
- moving two needles (2) to a coronary vessel of the animal; and
occluding the coronary vessel, partial or total, by moving the needles (2) together.
